(19) Europäisches Patentamt
European Patent Office
Office européen des brevets

(11) **EP 4 722 331 A1**

(12) **EUROPEAN PATENT APPLICATION**
published in accordance with Art. 153(4) EPC

(43) Date of publication:
08.04.2026 Bulletin 2026/15

(21) Application number: 24815364.5

(22) Date of filing: 23.05.2024

(51) International Patent Classification (IPC):
$C12M\ 1/00^{(2006.01)}$    $C12N\ 1/12^{(2026.01)}$

(52) Cooperative Patent Classification (CPC):
C12M 1/00; C12N 1/12

(86) International application number:
PCT/JP2024/019019

(87) International publication number:
WO 2024/247880 (05.12.2024 Gazette 2024/49)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 02.06.2023 JP 2023091622

(71) Applicant: Panasonic Intellectual Property
Management Co., Ltd.
Kadoma-shi, Osaka 571-0057 (JP)

(72) Inventors:
• KUSAMA, Shoko
Kadoma-shi, Osaka 571-0057 (JP)
• KOJIMA, Seiji
Kadoma-shi, Osaka 571-0057 (JP)

(74) Representative: Grünecker Patent- und
Rechtsanwälte
PartG mbB
Leopoldstraße 4
80802 München (DE)

(54) **CULTURE DEVICE FOR PROKARYOTIC PHOTOSYNTHETIC ORGANISMS AND METHOD FOR CULTURING PROKARYOTIC PHOTOSYNTHETIC ORGANISMS**

(57) A culture device 1a includes a container 10 and a light source 20. The container 10 is configured to accommodate a culture liquid $L_C$ containing prokaryotic photosynthetic organisms. The light source 20 is disposed inside the container 10. In the culture device 1a, conditions $(A \times B)/(C \times D) \geq 10 \times 10^3$ [$\mu$mol·m$^{-3}$·s$^{-1}$] and $B/D \geq 1.3 \times 10^{-1}$ [m$^{-1}$] are satisfied. A is a photon flux density [$\mu$mol·m$^{-2}$·s$^{-1}$] of the light source 20. B is a surface area [m$^2$] of the light source 20. C is an optical density [-] of the culture liquid $L_C$ accommodated in the container 10. D is a volume [m$^3$] of the culture liquid $L_C$ accommodated in the container 10.

FIG. 1

EP 4 722 331 A1

## Description

Technical Field

The present disclosure relates to a device for culturing prokaryotic photosynthetic organisms and a method for culturing prokaryotic photosynthetic organisms.

Background Art

Hitherto, culturing of prokaryotic photosynthetic organisms such as cyanobacteria has been attempted for the purpose of substance production.

For example, NPL 1 describes problems of mass culture of *Spirulina*, which is a filamentous cyanobacterium, on the basis of information obtained from two commercial facilities: Siam Algae Company and Earthrise Farms.

PTL 1 discloses an expandable vertical unit for culturing photosynthetic microorganisms. This vertical unit includes at least one sealable photobioreactor.

PTL 2 discloses a photosynthetic reactor suitable for culturing photosynthetic microorganisms, in particular, algae. This photosynthetic reactor includes a photosynthetic reaction pipe provided with at least one substantially horizontal reaction section.

PTL 3 discloses a panel for a photobioreactor. This panel includes two plates and two openings. At least one of the two plates is transparent, and a lighting device is disposed between the two plates. The lighting device is a textile including at least one optical fiber that can diffuse light through the at least one transparent plate.

Citation List

Patent Literature

PTL 1: Japanese Unexamined Patent Application Publication (Translation of PCT Application) No. 2022-517443
PTL 2: Japanese Unexamined Patent Application Publication (Translation of PCT Application) No. 2012-521209
PTL 3: Japanese Unexamined Patent Application Publication (Translation of PCT Application) No. 2020-511934

Non Patent Literature

NPL 1: Shimamatsu, Hidenori. "Mass production of Spirulina, an edible microalga." Hydrobiologia 512. 1-3 (2004): 39-44.

Summary of Invention

The present disclosure provides a culture device that is advantageous in terms of the efficiency and scale-up of the culture of prokaryotic photosynthetic organisms.

A device for culturing prokaryotic photosynthetic organisms according to the present disclosure includes

a container configured to accommodate a culture liquid containing prokaryotic photosynthetic organisms; and
a light source disposed inside the container,
wherein conditions $(A \times B)/(C \times D) \geq 10 \times 10^3$ [$\mu mol \cdot m^{-3} \cdot s^{-1}$] and $B/D \geq 1.3 \times 10^{-1}$ [$m^{-1}$] are satisfied, and
in the conditions,
A is a photon flux density [$\mu mol \cdot m^{-2} \cdot s^{-1}$] of the light source,
B is a surface area [$m^2$] of the light source,
C is an optical density [-] of the culture liquid accommodated in the container, and
D is a volume [$m^3$] of the culture liquid accommodated in the container.

The present disclosure can provide a culture device that is advantageous in terms of the efficiency and scale-up of the culture of prokaryotic photosynthetic organisms.

Brief Description of Drawings

[Fig. 1] Fig. 1 is a schematic view illustrating an example of a device for culturing prokaryotic photosynthetic organisms according to the present disclosure.

[Fig. 2] Fig. 2 is a flowchart schematically illustrating an example of a method for culturing prokaryotic photosynthetic organisms according to the present disclosure.

[Fig. 3] Fig. 3 is a graph showing the relationship between a specific growth rate of prokaryotic photosynthetic organisms and a photon parameter.

Description of Embodiments

(Underlying Knowledge Forming Basis of the Present Disclosure)

Methods for culturing prokaryotic photosynthetic organisms include an open-system method and a closed-system method. In the open-system method, a pond-like culture tank called an open pond is used. This method can be performed using a simple structure, and the culture tank is easy to handle. Therefore, the cost of the culture of prokaryotic photosynthetic organisms tends to be low. The culture of *Spirulina* described in NPL 1 is the culture using an open pond.

In the closed-system method, a vertical or horizontal tubular or panel-like culture container is used. In this method, the culture environment of prokaryotic photosynthetic organisms is easily adjusted, and the efficiency of culture tends to be high. In addition, the possibility of contamination tends to be low. It is understood that the reactors described in PTL 1 to PTL 3 are used in the closed-system method.

In the open-system culture method using an open pond, it is difficult to adjust the culture environment, such as temperature conditions and lighting conditions, and the efficiency of culture of prokaryotic photosynthetic organisms is less likely to be increased. On the other hand, in the existing closed-system method, the structure of the culture container tends to be complicated, making it difficult to achieve scale-up. For example, the volume of the culture container used in the existing closed-system method remains less than 100 liters.

In view of the circumstances described above, the present inventors have conducted extensive studies on whether it is possible to develop a culture device that can easily increase the efficiency of culture of prokaryotic photosynthetic organisms and that can be easily scaled up. As a result, it has been newly found that, in a culture device including a container configured to accommodate a culture liquid containing prokaryotic photosynthetic organisms and a light source disposed inside the container, when predetermined conditions related to the light source are satisfied, the efficiency of the culture of the prokaryotic photosynthetic organisms tends to be high. Based on this new finding, the culture device according to the present disclosure has been devised.

(Embodiments)

Embodiments of the present disclosure will be described below with reference to the drawings. The present disclosure is not limited to the following embodiments.

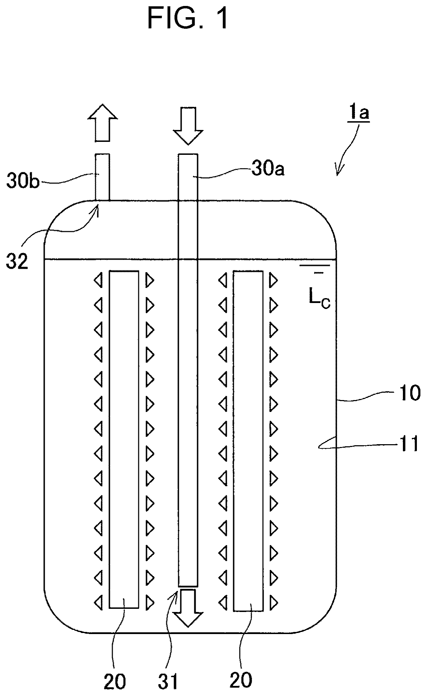

Fig. 1 is a schematic view illustrating an example of a device for culturing prokaryotic photosynthetic organisms. As illustrated in Fig. 1, a culture device 1a includes a container 10 and a light source 20. The container 10 is configured to accommodate a culture liquid $L_C$ containing prokaryotic photosynthetic organisms. The light source 20 is disposed inside the container 10. In the culture device 1a, conditions $(A \times B)/(C \times D) \geq 10 \times 10^3$ [$\mu$mol·m$^{-3}$·s$^{-1}$] and $B/D \geq 1.3 \times 10^{-1}$ [m$^{-1}$] are satisfied. In the conditions, A is a photon flux density [$\mu$mol·m$^{-2}$·s$^{-1}$] of the light source 20. The photon flux density A can be determined by, for example, performing measurement for a surface of the light source 20 using a commercially available quantum meter. The photon flux density A is, for example, a photosynthetic photon flux density, which is the number of photons per unit time and unit area within a wavelength range of 400 nm to 700 nm. B is a surface area [m$^2$] of the light source 20. In other words, B is the area of the light-emitting surface of the light source 20. C is an optical density (OD value) [-] of the culture liquid $L_C$ accommodated in the container 10. The optical density C is, for example, an optical density $OD_{730}$ at a wavelength of 730 nm. D is a volume [m$^3$] of the culture liquid $L_C$ accommodated in the container 10. Herein, $(A \times B)/(C \times D)$ is also referred to as a "photon parameter", and B/D is also referred to as a "specific light emission area parameter".

When the above conditions are satisfied in the culture device 1a, desired light energy is supplied to a wide range of the culture liquid $L_C$ accommodated inside the container 10, and prokaryotic photosynthetic organisms tend to be cultured at a high specific growth rate. As a result, the efficiency of the culture of the prokaryotic photosynthetic organisms tends to be high. In addition, since the above conditions are satisfied by adjusting the light source 20 and the culture liquid $L_C$, the culture of prokaryotic photosynthetic organisms is easily scaled up.

The upper limit of the photon parameter is not limited to a particular value as long as the above conditions are satisfied. The photon parameter is, for example, less than or equal to $50 \times 10^3$ [$\mu$mol·m$^{-3}$·s$^{-1}$]. In this case, photoinhibition tends to be suppressed.

From the viewpoint of the efficiency of culture of prokaryotic photosynthetic organisms, the photon parameter is desirably greater than or equal to $12 \times 10^3$ [$\mu$mol·m$^{-3}$·s$^{-1}$], more desirably greater than or equal to $15 \times 10^3$ [$\mu$mol·m$^{-3}$·s$^{-1}$],

and still more desirably greater than or equal to $20 \times 10^3$ [$\mu$mol·m$^{-3}$·s$^{-1}$]. From the viewpoint of suppressing photoinhibition, the photon parameter is desirably less than or equal to $40 \times 10^3$ [$\mu$mol·m$^{-3}$·s$^{-1}$], more desirably less than or equal to $30 \times 10^3$ [$\mu$mol·m$^{-3}$·s$^{-1}$], and still more desirably less than or equal to $25 \times 10^3$ [$\mu$mol·m$^{-3}$·s$^{-1}$]. The photon parameter may be greater than or equal to $12 \times 10^3$ [$\mu$mol·m$^{-3}$·s$^{-1}$], greater than or equal to $15 \times 10^3$ [$\mu$mol·m$^{-3}$·s$^{-1}$], or greater than or equal to $20 \times 10^3$ [$\mu$mol·m$^{-3}$·s$^{-1}$] and may be less than or equal to $50 \times 10^3$ [$\mu$mol·m$^{-3}$·s$^{-1}$], less than or equal to $40 \times 10^3$ [$\mu$mol·m$^{-3}$·s$^{-1}$], less than or equal to $30 \times 10^3$ [$\mu$mol·m$^{-3}$·s$^{-1}$], or less than or equal to $25 \times 10^3$ [$\mu$mol·m$^{-3}$·s$^{-1}$].

The upper limit of the specific light emission area parameter is not limited to a particular value as long as the above conditions are satisfied. The specific light emission area parameter is, for example, less than or equal to $50 \times 10^{-1}$ [m$^{-1}$]. In this case, the volume occupied by the light source 20 inside the container 10 is less likely to be large, and the volume of the culture liquid $L_C$ that can be accommodated in the container 10 is likely to be large.

From the viewpoint of the volume occupied by the light source 20 inside the container 10, the specific light emission area parameter is desirably less than or equal to $48 \times 10^{-1}$ [m$^{-1}$], more desirably less than or equal to $45 \times 10^{-1}$ [m$^{-1}$], and still more desirably less than or equal to $42 \times 10^{-1}$ [m$^{-1}$]. The specific light emission area parameter may be greater than or equal to $1.5 \times 10^{-1}$ [m$^{-1}$], greater than or equal to $2.0 \times 10^{-1}$ [m$^{-1}$], greater than or equal to $5.0 \times 10^{-1}$ [m$^{-1}$], greater than or equal to $10 \times 10^{-1}$ [m$^{-1}$], greater than or equal to $20 \times 10^{-1}$ [m$^{-1}$], greater than or equal to $30 \times 10^{-1}$ [m$^{-1}$], or greater than or equal to $40 \times 10^{-1}$ [m$^{-1}$] and may be less than or equal to $50 \times 10^{-1}$ [m$^{-1}$], less than or equal to $48 \times 10^{-1}$ [m$^{-1}$], or less than or equal to $42 \times 10^{-1}$ [m$^{-1}$].

The prokaryotic photosynthetic organisms contained in the culture liquid $L_C$ are not limited to particular prokaryotic photosynthetic organisms. The prokaryotic photosynthetic organisms are, for example, cyanobacteria. Cyanobacteria are also called blue-green algae or blue-green bacteria and are a group of prokaryotes that collect light energy with chlorophyll and split water with the obtained energy to produce oxygen, thus performing photosynthesis. Cyanobacteria are highly diverse. For example, in terms of the cell shape, there are unicellular species such as *Synechocystis* sp. PCC 6803 and filamentous species such as *Anabaena* sp. PCC 7120, in which multiple cells are connected. In terms of the growth environment, there are thermophilic species such as *Thermosynechococcus elongatus*, marine species such as *Synechococcus elongatus*, and freshwater species such as *Synechocystis.* There are also many species with unique characteristics such as *Microcystis aeruginosa*, which has gas vesicles and produces toxins, and *Gloeobacter violaceus*, which does not have thylakoids but has a protein called phycobilisome, which is a light-harvesting antenna, in the plasma membrane.

The container 10 is not limited to a particular container as long as prokaryotic photosynthetic organisms can be cultured. An inner surface 11 of the container 10 is made of, for example, metal. The inner surface 11 may be a mirror surface. In this case, light emitted from the light source 20 tends to be effectively utilized in the culture of prokaryotic photosynthetic organisms, and the efficiency of the culture of the prokaryotic photosynthetic organisms tends to be higher.

When the inner surface 11 of the container 10 is made of metal, the material forming the inner surface 11 may be an elemental metal or an alloy. An example of the alloy is stainless steel.

A volume $V_L$ of the culture liquid $L_C$ that can be accommodated in the container 10 is not limited to a particular value. The volume $V_L$ is, for example, greater than or equal to 0.4 m$^3$. In this case, the scale of the culture of prokaryotic photosynthetic organisms performed by the culture device 1a is easily increased.

The volume $V_L$ may be greater than or equal to 0.5 m$^3$, greater than or equal to 1 m$^3$, greater than or equal to 2 m$^3$, or greater than or equal to 5 m$^3$. The volume $V_L$ is, for example, less than or equal to 5 m$^3$.

The light source 20 is not limited to a particular light source as long as the above conditions are satisfied. The light source 20 emits light of at least one wavelength included in a wavelength range of 400 nm to 700 nm. In this case, the efficiency of the culture of the prokaryotic photosynthetic organisms tends to be higher. The culture device 1a may include a single light source 20 or light sources 20. That is, the number of light sources included in the culture device 1a may be one, or greater than or equal to two. The light sources 20 may be capable of emitting light of the same wavelength, or may be capable of emitting light of wavelengths different from each other.

As illustrated in Fig. 1, the light source 20 is, for example, disposed to be immersible in the culture liquid $L_C$. According to this configuration, the light from the light source 20 is applied to the culture liquid $L_C$ while the light source 20 is immersed in the culture liquid $L_C$. Thus, the light emitted from the light source 20 tends to be effectively utilized in the culture of prokaryotic photosynthetic organisms. As a result, the efficiency of the culture of the prokaryotic photosynthetic organisms tends to be higher.

At least part of the light source 20 is located, for example, below a horizontal plane that divides the inside of the container 10 equally into two portions on a volume basis. According to this configuration, the light from the light source 20 is easily applied while at least part of the light source 20 is immersed in the culture liquid $L_C$, and the light emitted from the light source 20 tends to be effectively utilized in the culture of prokaryotic photosynthetic organisms. As a result, the efficiency of the culture of the prokaryotic photosynthetic organisms tends to be higher.

The light-emitting surface of the light source 20 is not limited to a particular shape. The light-emitting surface of the light source 20 may be a flat surface, a curved surface, a cylindrical surface, or a spherical surface.

As illustrated in Fig. 1, the culture device 1a is provided with, for example, a supply port 31 and a discharge port

# EP 4 722 331 A1

32. The supply port 31 is disposed at a lower portion of the container 10, and air is introduced inside the container 10 through the supply port 31. The supply port 31 is located, for example, below the center of the container 10 in the direction of gravity. The supply port 31 is disposed, for example, near the bottom of the container 10. The discharge port 32 is disposed at an upper portion of the container 10. The discharge port 32 is located, for example, above the center of the container 10 in the direction of gravity. The air inside the container 10 is discharged to the outside of the container 10 through the discharge port 32. According to this configuration, air is introduced to a lower portion of the container 10, and the air is discharged from an upper portion of the container 10. Thus, the efficiency of the culture of the prokaryotic photosynthetic organisms tends to be higher.

The culture device 1a includes, for example, a supply pipe 30a and a discharge pipe 30b. The supply pipe 30a extends from the outside of the container 10 to the inside of the container 10, and one end of the supply pipe 30a located inside the container 10 forms the supply port 31. The discharge pipe 30b causes the inside of the container 10 to communicate with the outside of the container 10. The discharge pipe 30b has one end in contact with the inside of the container 10, and the one end forms the discharge port 32.

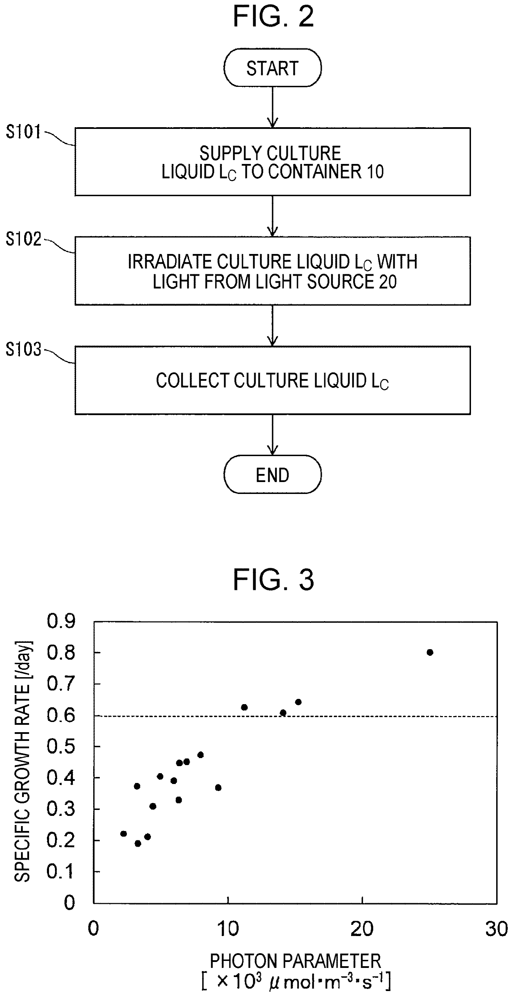

Fig. 2 is a flowchart schematically illustrating an example of a method for culturing prokaryotic photosynthetic organisms according to the present disclosure. This culture method includes irradiating a culture liquid $L_C$ containing prokaryotic photosynthetic organisms and accommodated in a container 10 with light from a light source 20 disposed inside the container 10 under the above-described conditions related to the photon parameter and the specific light emission area parameter. According to this culture method, the efficiency of the culture of the prokaryotic photosynthetic organisms tends to be high, and the culture of the prokaryotic photosynthetic organisms is easily scaled up.

As illustrated in Fig. 2, in step S101, a culture liquid $L_C$ is supplied to a container 10. Subsequently, in step S102, the culture liquid $L_C$ is irradiated with light from a light source 20 under the conditions described above to culture prokaryotic photosynthetic organisms. For example, the amount of culture liquid $L_C$ supplied to the container 10, the emission intensity of the light source 20, the number of light sources 20 that emit light, etc. are adjusted so as to satisfy the conditions described above. When the optical density OD of the culture liquid $L_C$ becomes greater than or equal to a predetermined value through the culture of the prokaryotic photosynthetic organisms, the light emission from the light source 20 is stopped, and the culture liquid $L_C$ is collected from the container 10 in step S103.

This completes a series of processes related to the culture of the prokaryotic photosynthetic organisms.

(Appendix)

The above descriptions disclose the following techniques.

(Technique 1)

A device for culturing prokaryotic photosynthetic organisms, including:

a container configured to accommodate a culture liquid containing prokaryotic photosynthetic organisms; and
a light source disposed inside the container,
wherein conditions $(A \times B)/(C \times D) \geq 10 \times 10^3$ [$\mu$mol·m$^{-3}$·s$^{-1}$)] and $B/D \geq 1.3 \times 10^{-1}$ [m$^{-1}$] are satisfied, and
in the conditions,
A is a photon flux density [$\mu$mol·m$^{-2}$·s$^{-1}$] of the light source,
B is a surface area [m$^2$] of the light source,
C is an optical density [-] of the culture liquid accommodated in the container, and
D is a volume [m$^3$] of the culture liquid accommodated in the container.

(Technique 2)

The culture device according to technique 1,
wherein the prokaryotic photosynthetic organisms are cyanobacteria.

(Technique 3)

The culture device according to technique 1 or 2,
wherein the container has an inner surface that is made of metal or that is a mirror surface.

(Technique 4)

The culture device according to any one of techniques 1 to 3, further including:

a supply port which is disposed at a lower portion of the container and through which air is introduced; and
a discharge port which is disposed at an upper portion of the container and through which the air is discharged.

(Technique 5)

The culture device according to any one of techniques 1 to 4,
wherein the light source is disposed to be immersible in the culture liquid.

(Technique 6)

The culture device according to any one of techniques 1 to 5,
wherein the light source emits light of at least one wavelength included in a wavelength range of 400 nm to 700 nm.

(Technique 7)

The culture device according to any one of techniques 1 to 6,
wherein the container is configured to accommodate the culture liquid with a volume of greater than or equal to 0.4 $m^3$.

(Technique 8)

A method for culturing prokaryotic photosynthetic organisms, including:

irradiating a culture liquid containing prokaryotic photosynthetic organisms and accommodated in a container with light from a light source disposed inside the container under conditions satisfying $(A \times B)/(C \times D) \geq 10 \times 10^3$ [$\mu$mol·m$^{-3}$·s$^{-1}$)] and $B/D \geq 1.3 \times 10^{-1}$ [m$^{-1}$],
wherein, in the conditions,
A is a photon flux density [$\mu$mol·m$^{-2}$·s$^{-1}$] of the light source,
B is a surface area [$m^2$] of the light source,
C is an optical density [-] of the culture liquid accommodated in the container, and
D is a volume [$m^3$] of the culture liquid accommodated in the container.

(Technique 9)

The culture method according to technique 8,
wherein the prokaryotic photosynthetic organisms are cyanobacteria.

(Technique 10)

The culture method according to technique 8 or 9,
wherein the container has an inner surface that is made of metal or that is a mirror surface.

(Technique 11)

The culture method according to any one of techniques 8 to 10,
wherein air is introduced into a lower portion of the container, and the air is discharged from an upper portion of the container.

(Technique 12)

The culture method according to any one of techniques 8 to 11,
wherein the culture liquid is irradiated with light from the light source while the light source is immersed in the culture liquid.

(Technique 13)

The culture method according to any one of techniques 8 to 12,
wherein light of at least one wavelength included in a wavelength range of 400 nm to 700 nm is emitted from the light source.

(Technique 14)

The culture method according to any one of techniques 8 to 13,
wherein the culture liquid has a volume of greater than or equal to 0.4 m$^3$.

EXAMPLES

Hereinafter, the present disclosure will be described in more detail with reference to Examples. The Examples described below are merely illustrative, and the present disclosure is not limited to the Examples described below.

*Synechocystis* sp. PCC6803 (hereinafter referred to as "*Synechocystis*"), which is a cyanobacterial species, was added to 0.4 m$^3$ of a BG-11 culture medium such that an initial bacterial cell concentration $OD_{730}$ was 0.02 to 0.05 to prepare a culture liquid of *Synechocystis*. The initial bacterial cell concentration $OD_{730}$ is an optical density of the culture liquid at a wavelength of 730 nm. Table 1 shows the concentrations of components in the BG-11 culture medium.

[Table 1]

| Component | Concentration [mg/L] |
|---|---|
| Ethylenediaminetetraacetic acid disodium salt dihydrate | 1 |
| Ammonium iron(III) citrate | 6 |
| Citric acid | 6 |
| $NaNO_3$ | 1500 |
| $MgSO_4$ | 75 |
| $K_2HPO_4$ | 39 |
| $CaCl_2$ | 28.6 |
| $H_3BO_3$ | 2.86 |
| $MnCl_2 \cdot 4H_2O$ | 1.81 |
| $ZnSO_4 \cdot 7H_2O$ | 0.22 |
| $CuSO_4 \cdot 5H_2O$ | 0.08 |
| $Na_2MoO_4 \cdot H_2O$ | 0.021 |
| $Co(NO_3)_2 \cdot 6H_2O$ | 0.0494 |
| HEPES (The pH was adjusted to 7.5 with KOH.) | 2383 |

The above culture liquid of *Synechocystis* was placed inside a tank made of stainless steel SUS 304 and having an inner volume of 0.5 m$^3$, and the culture of *Synechocystis* was performed for four days while the temperature inside the tank was maintained at 30°C. An LED underwater fish lamp, long white manufactured by Ks garage was disposed as a light source inside the tank. The power consumption of the light source was 60 W. In the culture of *Synechocystis*, the light source was caused to emit light while being completely immersed in the culture liquid. A dimmer manufactured by Ks garage was used to adjust the brightness of the light source. In the culture of *Synechocystis*, air was introduced from a supply port located near the bottom of the tank, and the air was discharged from a discharge port disposed at an upper portion of the tank. The amount of air supplied to the tank was adjusted to 0.4 m$^3$/min.

A quantum meter MQ-200 manufactured by Apogee Instruments, Inc. was used to measure the photon flux density A at a surface of the light source. Consequently, the photon flux density A was 3,590 [$\mu$mol·m$^{-2}$·s$^{-1}$]. The photon flux density A is the number of photons per unit time and unit area within a wavelength range of 400 nm to 700 nm. The product A × B of the photon flux density A and the surface area B [m$^2$] of the light source was determined. The surface area of a single light source was 272 cm$^2$. Accordingly, for example, when the number of light sources disposed inside the tank was 1, the product A × B was calculated to be 97.6 [$\mu$mol·s$^{-1}$].

As an indicator indicating the density of *Synechocystis* in the culture liquid, the optical density $OD_{730}$ of the culture liquid at a wavelength of 730 nm was used. The optical density $OD_{730}$ of the culture liquid was multiplied by the volume 0.4 m$^3$ of the culture liquid to indirectly calculate the number of *Synechocystis* in the culture liquid. The product A × B was divided by the product of the optical density $OD_{730}$ of the culture liquid and the volume of the culture liquid to determine the value of the photon parameter [$\mu$mol·m$^{-3}$·s$^{-1}$].

During the culture of *Synechocystis*, the optical density $OD_{730}$ of the culture liquid at a wavelength of 730 nm was measured over time, and a specific growth rate $\mu_N$ [/day] of *Synechocystis* was determined from a formula (1) below. In the formula (1), $OD_{730\_N}$ is an optical density of the culture liquid at a wavelength of 730 nm on the Nth day of the culture of *Synechocystis*, and $OD_{730\_N+1}$ is an optical density of the culture liquid at a wavelength of 730 nm on the N+1th day of the culture of *Synechocystis.* In the case of a 4-day culture, N is 1 to 5.

$$\mu_N = \ln(OD_{730\_N+1} - OD_{730\_N}) \qquad \text{Formula (1)}$$

Fig. 3 is a graph showing the relationship between the specific growth rate of prokaryotic photosynthetic organisms and the photon parameter in the above culture of *Synechocystis*. As shown in Fig. 3, when the photon parameter was greater than or equal to $10 \times 10^3$ [$\mu mol \cdot m^{-3} \cdot s^{-1}$], a specific growth rate of greater than or equal to 0.6 [/day] was exhibited. When the photon parameter was greater than or equal to $10 \times 10^3$ [$\mu mol \cdot m^{-3} \cdot s^{-1}$], 2 to 74 light sources were used. In this case, the value of the specific light emission area parameter determined by dividing the surface area of the light sources by the volume 0.4 $m^3$ of the culture liquid was in the range of $1.3 \times 10^{-1}$ [$m^{-1}$] to $50 \times 10^{-1}$ [$m^{-1}$].

It is considered that, in the above culture of *Synechocystis,* since the tank is made of stainless steel, light emitted from the light source and reaches the inner surface of the tank is reflected on the inner surface, and most of the light emitted from the light source can contribute to the photosynthetic reaction. As described above, it was suggested that the efficiency of the culture of *Synechocystis* be increased under the conditions of a photon parameter of greater than or equal to $10 \times 10^3$ [$\mu mol \cdot m^{-3} \cdot s^{-1}$] and a specific light emission area parameter of greater than or equal to $1.3 \times 10^{-1}$ [$m^{-1}$].

Industrial Applicability

According to the present disclosure, substance production through the culture of prokaryotic photosynthetic organisms on a commercial scale can be realized.

Reference Signs List

1a    culture device
10    container
11    inner surface
20    light source
31    supply port
32    discharge port
$L_C$    culture liquid

**Claims**

1.   A device for culturing prokaryotic photosynthetic organisms, comprising:

a container configured to accommodate a culture liquid containing prokaryotic photosynthetic organisms; and
a light source disposed inside the container,
wherein conditions $(A \times B)/(C \times D) \geq 10 \times 10^3$ [$\mu mol \cdot m^{-3} \cdot s^{-1}$)] and $B/D \geq 1.3 \times 10^{-1}$ [$m^{-1}$] are satisfied, and in the conditions,
A is a photon flux density [$\mu mol \cdot m^{-2} \cdot s^{-1}$] of the light source,
B is a surface area [$m^2$] of the light source,
C is an optical density [-] of the culture liquid accommodated in the container, and
D is a volume [$m^3$] of the culture liquid accommodated in the container.

2.   The culture device according to claim 1,
wherein the prokaryotic photosynthetic organisms are cyanobacteria.

3.   The culture device according to claim 1,
wherein the container has an inner surface that is made of metal or that is a mirror surface.

4.   The culture device according to claim 1, further comprising:

a supply port which is disposed at a lower portion of the container and through which air is introduced; and a discharge port which is disposed at an upper portion of the container and through which the air is discharged.

5. The culture device according to claim 1,
   wherein the light source is disposed to be immersible in the culture liquid.

6. The culture device according to claim 1,
   wherein the light source emits light of at least one wavelength included in a wavelength range of 400 nm to 700 nm.

7. The culture device according to claim 1,
   wherein the container is configured to accommodate the culture liquid with a volume of greater than or equal to 0.4 m$^3$.

8. A method for culturing prokaryotic photosynthetic organisms, comprising:

   irradiating a culture liquid containing prokaryotic photosynthetic organisms and accommodated in a container with light from a light source disposed inside the container under conditions satisfying $(A \times B)/(C \times D) \geq 10 \times 10^3$ [$\mu$mol·m$^{-3}$·s$^{-1}$] and $B/D \geq 1.3 \times 10^{-1}$ [m$^{-1}$],
   wherein, in the conditions,
   A is a photon flux density [$\mu$mol·m$^{-2}$·s$^{-1}$] of the light source,
   B is a surface area [m$^2$] of the light source,
   C is an optical density [-] of the culture liquid accommodated in the container, and
   D is a volume [m$^3$] of the culture liquid accommodated in the container.

9. The culture method according to claim 8,
   wherein the prokaryotic photosynthetic organisms are cyanobacteria.

10. The culture method according to claim 8,
    wherein the container has an inner surface that is made of metal or that is a mirror surface.

11. The culture method according to claim 8,
    wherein air is introduced into a lower portion of the container, and the air is discharged from an upper portion of the container.

12. The culture method according to claim 8,
    wherein the culture liquid is irradiated with light from the light source while the light source is immersed in the culture liquid.

13. The culture method according to claim 8,
    wherein light of at least one wavelength included in a wavelength range of 400 nm to 700 nm is emitted from the light source.

14. The culture method according to claim 8,
    wherein the culture liquid has a volume of greater than or equal to 0.4 m$^3$.

# FIG. 1

# FIG. 2

```
        ( START )
            │
            ▼
┌───────────────────────────────┐
│        SUPPLY CULTURE          │  S101
│  LIQUID L_C TO CONTAINER 10    │
└───────────────────────────────┘
            │
            ▼
┌───────────────────────────────┐
│ IRRADIATE CULTURE LIQUID L_C   │  S102
│  WITH LIGHT FROM LIGHT SOURCE  │
│              20                │
└───────────────────────────────┘
            │
            ▼
┌───────────────────────────────┐
│    COLLECT CULTURE LIQUID L_C  │  S103
└───────────────────────────────┘
            │
            ▼
         ( END )
```

# FIG. 3

## INTERNATIONAL SEARCH REPORT

| International application No. |
|---|
| **PCT/JP2024/019019** |

| A. | CLASSIFICATION OF SUBJECT MATTER |
|---|---|

*C12M 1/00*(2006.01)i; *C12N 1/12*(2006.01)i
FI:   C12M1/00 E; C12N1/12 A

According to International Patent Classification (IPC) or to both national classification and IPC

| B. | FIELDS SEARCHED |
|---|---|

Minimum documentation searched (classification system followed by classification symbols)

C12M1/00-1/42; C12N1/00-1/38

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); PubMed

| C. | DOCUMENTS CONSIDERED TO BE RELEVANT |
|---|---|

| Category* | Citation of document, with indication, where appropriate, of the relevant passages | Relevant to claim No. |
|---|---|---|
| Y | JP 2022-134906 A (PANASONIC INTELLECTUAL PROPERTY MANAGEMENT CO., LTD.) 15 September 2022 (2022-09-15)<br>  paragraphs [0152], [0159], [0171], [0172] | 1-14 |
| Y | JP 10-098964 A (SANYO ELECTRIC CO., LTD., TOTTORI SANYO ELECTRIC CO., LTD.) 21 April 1998 (1998-04-21)<br>  paragraph [0007], fig. 1 | 1-14 |
| Y | JP 2002-000256 A (SANYO ELECTRIC CO., LTD., TOTTORI SANYO ELECTRIC CO., LTD.) 08 January 2002 (2002-01-08)<br>  fig. 1 | 1-14 |
| Y | JP 2010-530738 A (WACKER CHEMIE AKTIENGESELLSCHAFT) 16 September 2010 (2010-09-16)<br>  fig. 1 | 1-14 |
| Y | JP 2017-000100 A (ASAHI KASEI KABUSHIKI KAISHA) 05 January 2017 (2017-01-05)<br>  claim 12 | 1-14 |

☑ Further documents are listed in the continuation of Box C.          ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
|---|---|
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **01 August 2024** | **13 August 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

# EP 4 722 331 A1

## INTERNATIONAL SEARCH REPORT

International application No.

**PCT/JP2024/019019**

### C. DOCUMENTS CONSIDERED TO BE RELEVANT

| Category* | Citation of document, with indication, where appropriate, of the relevant passages | Relevant to claim No. |
|---|---|---|
| Y | JP 2021-058134 A (MEIJI UNIVERSITY) 15 April 2021 (2021-04-15) paragraph [0018] | 1-14 |
| Y | WO 2018/043146 A1 (SHOWA DENKO K.K.) 08 March 2018 (2018-03-08) claim 6 | 1-14 |
| A | LUIMSTRA, V. M. et al. Blue light reduces photosynthetic efficiency of cyanobacteria through an imbalance between photosystems I and II. PHOTOSYNTHESIS RESEARCH. 2018. vol. 138, pp. 177-189 entire text | 1-14 |

Form PCT/ISA/210 (second sheet) (July 2022)

## INTERNATIONAL SEARCH REPORT
### Information on patent family members

International application No.

**PCT/JP2024/019019**

| Patent document cited in search report | | | Publication date (day/month/year) | Patent family member(s) | | | Publication date (day/month/year) |
|---|---|---|---|---|---|---|---|
| JP | 2022-134906 | A | 15 September 2022 | (Family: none) | | | |
| JP | 10-098964 | A | 21 April 1998 | (Family: none) | | | |
| JP | 2002-000256 | A | 08 January 2002 | (Family: none) | | | |
| JP | 2010-530738 | A | 16 September 2010 | US fig. 1<br>WO<br>EP<br>CN<br>KR | 2010/0190227<br>2008/145719<br>2150609<br>101679930<br>10-2010-0017975 | A1<br>A1<br>A1<br>A<br>A | |
| JP | 2017-000100 | A | 05 January 2017 | (Family: none) | | | |
| JP | 2021-058134 | A | 15 April 2021 | (Family: none) | | | |
| WO | 2018/043146 | A1 | 08 March 2018 | US claim 6<br>EP<br>CN | 2019/0223397<br>3508566<br>109642203 | A1<br>A1<br>A | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2022517443 W
- JP 2012521209 W

- JP 2020511934 W

**Non-patent literature cited in the description**

- **SHIMAMATSU** ; **HIDENORI**. Mass production of Spirulina, an edible microalga. *Hydrobiologia*, 2004, vol. 512 (1-3), 39-44